(19) Europäisches Patentamt
European Patent Office
Office européen des brevets

(11) EP 1 488 778 B1

(12) FASCICULE DE BREVET EUROPEEN

(45) Date de publication et mention de la délivrance du brevet:
**05.12.2007 Bulletin 2007/49**

(51) Int Cl.:
***A61K 8/31*** *(2006.01)* ***A61K 8/90*** *(2006.01)*
***A61Q 5/06*** *(2006.01)*

(21) Numéro de dépôt: **04291390.5**

(22) Date de dépôt: **04.06.2004**

(54) **Composition cosmétique capillaire à base d'isoeicosane et de polyuréthane fixant siliconé.**

Haarkosmetische Zusammensetzung, auf der Basis von Isoeicosan und einem siliconierten fixierenden Polyurethan.

A hair-treatment composition based on Isoeicosane and silicone fixing Polyurethane.

(84) Etats contractants désignés:
**AT BE BG CH CY CZ DE DK EE ES FI FR GB GR HU IE IT LI LU MC NL PL PT RO SE SI SK TR**

(30) Priorité: **17.06.2003 FR 0307289**

(43) Date de publication de la demande:
**22.12.2004 Bulletin 2004/52**

(73) Titulaire: **L'ORÉAL**
**75008 Paris (FR)**

(72) Inventeurs:
- **Vrignaud, Sabine**
**93200 La Plaine Saint Denis (FR)**
- **Belli, Emmanuelle**
**92600 Asnieres (FR)**

(74) Mandataire: **Zapalowicz, Francis**
**Bureau Casalonga & Josse**
**Bayerstrasse 71/73**
**80335 München (DE)**

(56) Documents cités:
**WO-A-97/12585**
**FR-A- 2 708 199**
**FR-A- 2 815 853**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 488 778 B1

## Description

La présente invention est relative à de nouvelles compositions cosmétiques capillaires comprenant de l'isoeicosane et au moins un polyuréthane fixant siliconé. Elle vise également un procédé cosmétique, en particulier un procédé de fixation et/ou de maintien de la coiffure mettant en oeuvre lesdites compositions, ainsi que l'utilisation de ces compositions pour conférer de la brillance aux cheveux.

Il est connu d'utiliser l'isoeicosane, une huile hydrocarbonée non volatile, comme agent de conditionnement dans des compositions cosmétiques capillaires.

Ainsi, la demande de brevet FR 2 815 853 décrit des compositions cosmétiques capillaire se présentant sous forme de mousse et comprenant au moins un polymère squelette aminoplaste, avec comme agent de conditionnement possible l'isoeicosane.

Toutefois, il existe un besoin de disposer de produits capillaires permettant de conférer aux cheveux du maintien et un aspect brillant durable, sans apport de caractère gras trop marqué. Ces propriétés ne sont pas apportées par les compositions de l'état de la technique.

La présente invention se propose de fournir une solution à cette attente.

En particulier, la Demanderesse vient de découvrir de façon surprenante que des compositions comprenant de l'isoeicosane et au moins un polyuréthane fixant siliconé ne présentaient pas l'ensemble des inconvénients indiqués ci-dessus, et en particulier fixaient bien la coiffure, tout en procurant de bonnes propriétés cosmétiques.

Elle a notamment constaté que ces compositions adhéraient bien aux cheveux et que les cheveux étaient particulièrement lisses au toucher.

Elle a en outre constaté que ces compositions conféraient aux cheveux une brillance intense et persistante.

Enfin, elle a découvert que ces compositions pouvaient être formulées en milieu alcoolique.

D'autres objets de l'invention apparaîtront à la lecture de la description et des exemples qui suivent.

La présente invention a donc pour objet une composition cosmétique capillaire, caractérisée en ce qu'elle comprend dans un milieu cosmétiquement acceptable, de l'isoeicosane et au moins un polyuréthane fixant siliconé.

Un autre objet de l'invention concerne un procédé cosmétique capillaire, en particulier un procédé de fixation et/ou de maintien de la coiffure mettant en oeuvre les compositions selon l'invention.

L'invention a également pour objet l'utilisation des compositions selon l'invention pour conférer de la brillance aux cheveux.

L'isoeicosane est un hydrocarbure liquide à température ambiante. Il peut notamment être vendu sous le nom PERMETHYL 102 A par la société Bayer.

L'isoeicosane est présent dans la composition à des teneurs comprises de préférence entre 0,05 et 20% en poids du poids total de la composition, et encore de préférence entre 1 et 10% en poids du poids total de la composition.

Au sens de la présente invention, on entend par polymère fixant tout polymère permettant de donner une forme à la chevelure ou de maintenir cette forme.

Les polyuréthanes fixants siliconés de l'invention peuvent être amphotères, anioniques ou non ioniques, et ils sont de préférence anioniques ou non ioniques.

De tels polymères sont par exemple décrits dans les brevets FR 2 7081 99 et FR 2 743 297.

A titre d'exemple, le polyuréthane fixant siliconé peut être formé par un arrangement de blocs, cet arrangement étant obtenu notamment à partir de :

(1) au moins un composé qui contient deux ou plus de deux atomes d'hydrogène actifs par molécule ;
(2) au moins un diol ou un mélange de diols contenant des fonctions acides ou leurs sels ;
(3) au moins un di- ou polyisocyanate ; et
(4) au moins un composé ayant un squelette siliconé.

Avantageusement, les composés (1) sont choisis dans le groupe comprenant les diols, les diamines, les polyesterols, les polyétherols ou leurs mélanges.

Les composés (1) qui sont préférés sont les polyéthylène-glycols et les polypropylèneglycols linéaires, en particulier ceux qui sont obtenus par réaction de l'oxyde d'éthylène ou de propylène avec l'eau ou du diéthylène ou du dipropylèneglycol en présence d'hydroxyde de sodium en tant que catalyseur. Ces polyalkylène-glycols ont généralement une masse moléculaire comprise entre environ 600 et 20 000.

D'autres composés organiques préférés sont ceux qui ont des groupes mercapto, amino, carboxyle ou hydroxyle. Parmi ceux-ci, on cite plus particulièrement les composés polyhydroxylés tels que les polyéther-diols, les polyester-diols, les polyacétal-diols, les polyamide-diols, les polyester-polyamide-diols, les poly(alkylène éther)-diols, les polythioéther-diols et les polycarbonate-diols.

Les polyéther-diols préférés sont, par exemple, les produits de condensation d'oxyde d'éthylène, d'oxyde de propylène ou de tétrahydrofurane, leurs produits de copolymérisation ou de condensation, greffés ou blocs, tels que les

mélanges de condensats d'oxyde d'éthylène et de propylène, et les produits de polymérisation d'oléfines, sous haute pression, avec les condensats d'oxyde d'alkylène. Des polyéthers appropriés sont par exemple préparés par condensation d'oxydes d'alkylène et d'alcools polyhydriques, tels que l'éthylèneglycol, le 1,2-propylèneglycol et le 1,4-butanediol.

Les polyester-diols, polyester-amides, polyamide-diols sont de préférence saturés et sont obtenus, par exemple, à partir de la réaction d'acides polycarboxyliques saturés ou insaturés avec des alcools polyhydriques, des diamines ou des polyamines. Pour préparer ces composés, on peut utiliser, par exemple, l'acide adipique, l'acide succinique, l'acide phtalique, l'acide téréphtalique et l'acide maléique. Des alcools polyhydriques appropriés pour préparer les polyesters incluent par exemple l'éthylèneglycol, le 1,2-propylèneglycol, le 1,4-butanediol, le néopentylglycol et l'hexanediol. On peut aussi utiliser des aminoalcools, par exemple l'éthanolamine. Des diamines appropriées pour préparer les amide-polyesters sont l'éthylène-diamine et l'hexaméthylène-diamine.

Des polyacétals appropriés peuvent être préparés, par exemple, à partir de 1,4-butanediol ou d'hexanediol et de formaldéhyde. Des polythioéthers appropriés peuvent être préparés par exemple par réaction de condensation entre des thioglycols seuls ou en combinaison avec d'autres glycols tels que l'éthylèneglycol, le 1,2-propylèneglycol ou avec d'autres composés polyhydroxylés. Les composés polyhydroxylés contenant déjà des groupements uréthannes, des polyols naturels, qui peuvent être davantage modifiés, par exemple, l'huile de castor et les carbohydrates peuvent également être utilisés.

Plus préférentiellement, le composé du groupe (1) est un polyestérol, notamment un polyester-diol formé par la réaction d'au moins un (di)-polyol ($1_a$) et d'au moins un acide ($1_b$). Le (di)- polyol ($1_a$) est en particulier choisi dans le groupe comprenant le néopentylglycol, le 1,4-butanediol, l'hexanediol, l'éthylèneglycol, le diéthylèneglycol, le propylèneglycol, le butylèneglycol, le néopentylglycol et (di)-polyéthylèneglycol. L'acide ($1_b$) est en particulier choisi dans le groupe comprenant l'acide phtalique, l'acide isophtalique, l'acide adipique et l'acide (poly)-lactique.

En tant que composé (2), on peut notamment utiliser un acide hydroxycarboxylique tel que l'acide diméthylolpropanoïque (DMPA) ou un acide 2,2-hydroxyméthyl-carboxylique. En général, le composé (2) est utile en tant que bloc de couplage. En tant que composés (2), on préfère ceux comprenant au moins un poly (acide-(alpha-hydroxycarboxyliquediol)).

Les composés (2) particulièrement préférés conformément à l'invention sont ceux choisis dans le groupe comprenant l'acide 2,2-di(hydroxyméthyl)acétique, l'acide 2,2-dihydroxyméthylpropionique, l'acide 2,2-dihydroxyméthylbutyrique, l'acide 2,2-dihydroxyméthylpentanoïque.

Le di- ou polyisocyanate (3) peut être choisi en particulier dans le groupe comprenant l'hexaméthylène-diisocyanate, l'isophorone-diisocyanate (IDPI), le toluylène-diisocyanate, le diphénylméthane-4,4'-diisocyanate (DPMD) et le dicyclohexylméthane-4,4'-diisocyanate (DCMD), le méthylène-di-p-phényl-diisocyanate, le méthylène-bis(4-cyclohexylisocyanate), les toluène-diisocyanates, le 1,5-naphtalène-diisocyanate, le 4,4'-diphénylméthane-diisocyanate, le 2,2'-diméthyl-4,4'-diphénylméthane-diisocyanate, le 1,3-phénylène-diisocyanate, le 1,4-phénylène diisocyanate, des mélanges de 2,4- et de 2,6-toluène-diisocyanates, le 2,2'-dichloro-4,4'-diisocyanato-diphénylméthane, le 2,4-dibromo-1,5-diisocyanato naphtalène, le butane-1,4-diisocyanate, l'hexane-1,6-diisocyanate, le cyclohexane-1,4-diisocyanate.

Le composé ayant un squelette siliconé (4) peut être choisi parmi les polysiloxanes, les polyalkylsiloxanes ou les polyarylsiloxanes, notamment les polyéthylsiloxanes, les polyméthylsiloxanes et les polyphénylsiloxanes, comportant éventuellement des chaînes hydrocarbonées greffées sur les atomes de silicium.

Le polyuréthane fixant peut être formé à l'aide d'un composé supplémentaire (5) servant en général à allonger sa chaîne. Ces composés (5) peuvent être choisis dans le groupe comprenant notamment les glycols saturés ou insaturés tels que l'éthylèneglycol, le diéthylèneglycol, le néopentylglycol, le triéthylène glycol ; les aminoalcools tels que l'éthanolamine, la propanolamine, la butanolamine ; les amines primaires hétérocycliques, aromatiques, cycloaliphatiques, et aliphatiques ; les diamines ; les acides carboxyliques tels que les acides carboxyliques aliphatiques, aromatiques et hétérocycliques comme les acides oxalique, succinique, glutarique, adipique, sébacique et téréphtalique ; les acides aminocarboxyliques. Les composés (5) préférés sont les diols aliphatiques.

Le polyuréthane fixant siliconé utilisé dans la présente invention peut avantageusement comprendre au moins une séquence polysiloxane et son motif répétitif de base répond par exemple à la formule générale (II) :

$$- O - P - O - CO - NH - R - NH - CO - \qquad (II)$$

dans laquelle :

- P est un segment polysiloxanique, et
- R est un groupe divalent choisi parmi les groupes alkylènes de type aromatique, aliphatique en $C_1$ à $C_{20}$, cycloaliphatique en $C_1$ à $C_{20}$, ces groupes étant substitués ou non.

Avantageusement, le segment polysiloxanique P répond à la formule générale (III) ci-après :

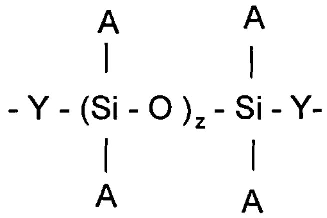

( III )

dans laquelle:

- les groupes A, qui peuvent être identiques ou différents, sont choisis parmi d'une part les groupes hydrocarbonés monovalents en $C_1$ à $C_{20}$ exempts ou substantiellement exempts d'insaturation éthylénique et, d'autre part, les groupes aromatiques,
- Y représente un groupe hydrocarboné divalent, et
- z représente un nombre entier, choisi de telle sorte que la masse moléculaire moyenne du segment polysiloxane soit comprise entre 300 et 10 000.

En général, le groupe divalent Y est choisi parmi les groupes alkylènes de formule $-(CH_2)_a-$ , dans laquelle a représente un nombre entier pouvant être compris entre 1 et 10.

Les groupes A peuvent être choisis parmi les groupes alkyles, en particulier les groupes méthyle, éthyle, propyle, isopropyle, butyle, pentyle, hexyle, octyle, décyle, dodécyle et octadécyle ; les groupes cycloalkyle, en particulier le groupe cyclohexyle ; les groupes aryle, notamment phényle et naphtyle ; les groupes arylalkyle, notamment benzyle et phényléthyle, ainsi que les groupes tolyle et xylyle.

A titre d'exemples de polyuréthane fixant siliconé, on peut notamment citer le copolymère acide diméthylol-propionique/isophorone-diisocyanate/néopentylglycol/polyesterdiols/ diamine siliconé (connu aussi sous le nom de polyuréthane-6, appellation INCI) vendu sous la marque Luviset® Si PUR par la société BASF.

Le polyuréthane fixant siliconé est de préférence présent dans la composition à des teneurs comprises entre 0,01 et 20 % en poids, encore plus préférentiellement entre 0,05 et 15 %, et mieux encore entre 0,1 et 10% en poids du poids total de la composition.

Les compositions selon l'invention peuvent également comprendre un ou plusieurs polymères fixants additionnels différents des polyuréthanes fixants siliconés décrits ci-dessus.

Les polymères fixants additionnels convenant dans l'invention sont notamment choisis parmi les polymères cationiques, anioniques, amphotères, non ioniques et leurs mélanges.

Les polymères fixants additionnels cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant une masse moléculaire moyenne en nombre comprise entre 500 et environ 5 000 000, et de préférence entre 1 000 et 3 000 000.

Parmi ces polymères, on peut citer plus particulièrement les polymères cationiques suivants :

(1) les homopolymères ou copolymères d'esters ou d'amides acryliques ou méthacryliques, à fonctions aminées, comportant au moins un des motifs de formules suivantes :

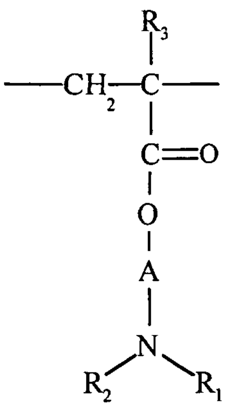 (A) , 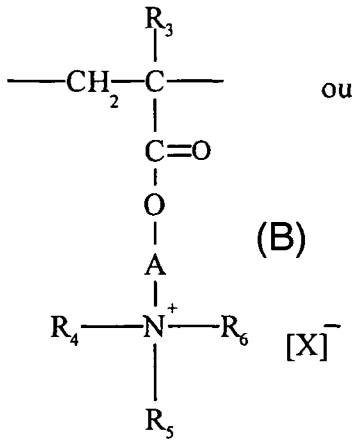 (B) ou 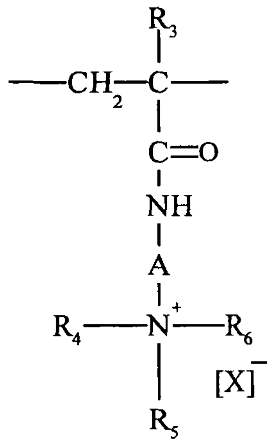 (C)

dans lesquelles:

$R_1$ et $R_2$, identiques ou différents, représentent chacun un atome d'hydrogène ou un groupe alkyle ayant de 1 à 6 atomes de carbone ;
$R_3$ désigne un atome d'hydrogène ou un groupe $CH_3$ ;
A est un groupe alkyle linéaire ou ramifié, comportant de 1 à 6 atomes de carbone ou un groupe hydroxyalkyle comportant de 1 à 4 atomes de carbone ;
$R_4$, $R_5$, $R_6$, identiques ou différents, représentent un groupe alkyle ayant de 1 à 18 atomes de carbone ou un groupe benzyle ;
X désigne un anion méthosulfate ou un halogénure tel que chlorure ou bromure.

Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieur ($C_{1-4}$), des groupes dérivés des acides acryliques ou méthacryliques ou de leurs esters, de vinyllactames tels que la vinylpyrrolidone ou le vinylcaprolactame, d'esters vinyliques.
Ainsi, parmi ces copolymères de la famille (1), on peut citer :

- les copolymères d'acrylamide et de méthacrylate de diméthylaminoéthyle quaternisés au sulfate de diméthyle ou avec un halogénure de diméthyle, tels que celui vendu sous la dénomination HERCOFLOC® par la société HERCULES,
- les copolymères d'acrylamide et de chlorure de méthacryloyloxyéthyltriméthylammonium décrits, par exemple, dans la demande de brevet EP-A-080976 et vendus sous la dénomination BINA QUAT P 100 par la société CIBA GEIGY,
- les copolymères d'acrylamide et de méthosulfate de méthacryloyloxyéthyltriméthylammonium, tels que celui vendu sous la dénomination RETEN par la société HERCULES,
- les copolymères vinylpyrrolidone/acrylate ou méthacrylate de dialkylaminoalkyle quaternisés ou non, tels que les produits vendus sous la dénomination "GAFQUAT®" par la société ISP comme, par exemple, "GAFQUAT® 734" ou "GAFQUAT® 755", ou bien les produits dénommés "COPOLYMER® 845, 958 et 937". Ces polymères sont décrits en détail dans les brevets français Nos 2 077 143 et 2 393 573,
- les polymères à chaîne grasse et à motif vinylpyrrolidone, tels que les produit vendus sous la dénomination Stylèze W20 et Stylèze W10 par la société ISP,
- les terpolymères méthacrylate de diméthylaminoéthyle/vinylcaprolactame/vinylpyrrolidone tels que le produit commercialisé sous la dénomination GAFFIX® VC 713 par la société ISP, et
- les copolymères vinylpyrrolidone/méthacrylamide de diméthylaminopropyle quaternisé tels que notamment le produit commercialisé sous la dénomination "GAFQUAT® HS 100" par la société ISP ;

(2) les polysaccharides cationiques, de préférence à ammonium quaternaire, tels que ceux décrits dans les brevets américains 3 589 578 et 4 031 307 tel que les gommes de guar contenant des groupements cationiques trialkylammonium. De tels produits sont commercialisés notamment sous les dénominations commerciales de JAGUAR C13 S, JAGUAR C 15, JAGUAR C 17 par la société MEYHALL;
(3) les copolymères quaternaires de vinylpyrrolidone et de vinylimidazole ;

(4) les chitosanes ou leurs sels ; les sels utilisables sont en particulier l'acétate, le lactate, le glutamate, le gluconate ou le pyrrolidone-carboxylate de chitosane.
Parmi ces composés, on peut citer le chitosane ayant un taux de désacétylation de 90,5 % en poids vendu sous la dénomination KYTAN BRUT STANDARD par la société ABER TECHNOLOGIES, le pyrrolidone-carboxylate de chitosane commercialisé sous la dénomination KYTAMER® PC par la société AMERCHOL.
(5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyloxyéthyl-triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Les polymères fixants additionnels anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono- ou diacides carboxyliques insaturés tels que ceux répondant à la formule :

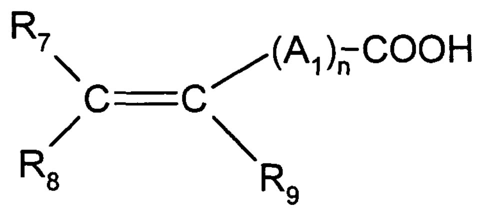

(I)

dans laquelle n est un nombre entier de 0 à 10, $A_1$ désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, $R_7$ désigne un atome d'hydrogène, un groupement phényle ou benzyle, $R_8$ désigne un atome d'hydrogène, un groupement alkyle inférieur ou carboxyle, $R_9$ désigne un atome d'hydrogène, un groupement alkyle inférieur, un groupement $-CH_2-COOH$, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :

A) Les homo- ou copolymères d'acide acrylique ou méthacrylique ou leurs sels et en particulier les produits vendus sous les dénominations VERSICOL® E ou K par la société ALLIED COLLOID, et ULTRAHOLD® par la société BASF, les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois n$^{os}$ 75370 et 75371 ou proposés sous la dénomination QUADRAMER par la Société AMERICAN CYANAMID. On peut également citer les copolymères d'acide acrylique et de méthacrylate d'alkyle en $C_1$-$C_4$ et les terpolymères de vinylpyrrolidone, d'acide acrylique et de méthacrylate d'alkyle en $C_1$-$C_{20}$, par exemple, de lauryle, tels que celui commercialisé par la société ISP sous la dénomination ACRYLIDONE® LM et les terpolymères acide méthacrylique/acrylate d'éthyle/acrylate de tertiobutyle tels que le produit commercialisé sous la dénomination LUVIMER® 100 P par la société BASF.
On peut aussi citer les copolymères acide méthacrylique/acide acrylique/acrylate d'éthyle/méthacrylate de méthyle en dispersion aqueuse, commercialisé sous la dénomination AMERHOLD® DR 25 par la société AMERCHOL.
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou

ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les brevets français n[os] 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch.

D) Les copolymères d'acides ou d'anhydrides carboxyliques monoinsaturés en $C_4$-$C_8$ choisis parmi :

- les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n[os] 2 047 398, 2 723 248, 2 102 113, le brevet GB n° 839 805. Des produits commerciaux sont notamment ceux vendus sous les dénominations GANTREZ® AN ou ES par la société ISP.
- les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,

les fonctions anhydrides de ces copolymères étant éventuellement monoestérifiées ou monoamidifiées.

Ces polymères sont par exemple décrits dans les brevets français nos 2 350 384 et 2 357 241 de la demanderesse.

E) Les polyacrylamides comportant des groupements carboxylates.

Les homopolymères et copolymères comprenant des groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :

- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
- les sels de l'acide polystyrène-sulfonique tels que les sels de sodium vendus par exemple sous les dénominations Flexan® 500 et Flexan® 130 par National Starch. Ces composés sont décrits dans le brevet FR 2 198 719.
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER HSP 1180 par Henkel.

Comme autre polymère fixant additionnels anionique utilisable selon l'invention, on peut citer le polymère anionique séquencé branché vendu sous la dénomination FIXATE G100 par la société NOVEON.

Selon l'invention, les polymères fixants additionnels anioniques sont de préférence choisis parmi les copolymères d'acide acrylique tels que les terpolymères acide acrylique/acrylate d'éthyle/N-tertiobutylacrylamide vendus notamment sous la dénomination ULTRAHOLD® STRONG par la société BASF, les copolymères dérivés d'acide crotonique tels que les terpolymères acétate de vinyle/tertiobutylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ® par la société ISP, les copolymères d'acide méthacrylique et de méthacrylate de méthyle vendus sous la dénomination EUDRAGIT® L par la société ROHM PHARMA, les copolymères d'acide méthacrylique et d'acrylate d'éthyle vendus sous la dénomination LUVIMER® MAEX ou MAE par la société BASF et les copolymères acétate de vinyle/acide crotonique vendus notamment sous la dénomination LUVISET CA 66 par la société BASF et les copolymères acétate de vinyle/acide crotonique greffés par du polyéthylèneglycol vendus sous la dénomination ARISTOFLEX® A par la société BASF, le polymère vendu sous la dénomination FIXATE G100 par la société NOVEON.

Parmi les polymères fixants additionnels anioniques cités ci-dessus, on préfère plus particulièrement utiliser dans le cadre de la présente invention les copolymères méthylvinyléther/anhydride maléique monoestérifiés vendus sous la dénomination GANTREZ® ES 425 par la société ISP, les terpolymères acide acrylique/acrylate d'éthyle/N-tertiobutylacrylamide vendus sous la dénomination ULTRAHOLD® STRONG par la société BASF, les copolymères d'acide méthacrylique et de méthacrylate de méthyle vendus sous la dénomination EUDRAGIT® L par la société ROHM

PHARMA, les terpolymères acétate de vinyle/tertio-butylbenzoate de vinyle/acide crotonique et les terpolymères acide crotonique/acétate de vinyle/néododécanoate de vinyle vendus sous la dénomination Résine 28-29-30 par la société NATIONAL STARCH, les copolymères d'acide méthacrylique et d'acrylate d'éthyle vendus sous la dénomination LUVIMER® MAEX OU MAE par la société BASF, les terpolymères vinylpyrrolidone/acide acrylique/méthacrylate de lauryle vendus sous la dénomination ACRYLIDONE® LM par la société ISP, le polymère vendu sous la dénomination FIXATE G100 par la société NOVEON.

Les polymères fixants additionnels amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-$\alpha,\beta$-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :

(1) les copolymères à motifs vinyliques acides et à motifs vinyliques basiques, tels que ceux résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les méthacrylate et acrylate de dialkylaminoalkyle, les dialkylaminoalkylméthacrylamide et acrylamide. De tels composés sont décrits dans le brevet américain n° 3 836 537.

(2) les polymères comportant des motifs dérivant :

a) d'au moins un monomère choisi parmi les acrylamides ou les méthacrylamides substitués sur l'atome d'azote par un groupe alkyle,

b) d'au moins un comonomère acide contenant un ou plusieurs groupements carboxyliques réactifs, et

c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les composés dont les groupes alkyle comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.

Les comonomères basiques préférés sont des méthacrylates d'aminoéthyle, de butylaminoéthyle, de N,N'-diméthylaminoéthyle, de N-tertio-butylaminoéthyle.

On utilise particulièrement les copolymères dont la dénomination CTFA (4ème Ed., 1991) est Octylacrylamide/acrylates/butylaminoethyl methacrylate copolymer, tels que les produits vendus sous la dénomination AMPHOMER® ou LOVOCRYL® 47 par la société NATIONAL STARCH.

(3) les polyaminoamides réticulés et acylés partiellement ou totalement dérivant de poyaminoamides de formule générale :

$$-\!\!\left[CO-R_{10}-CO-Z\right]\!\!- \qquad \text{(II)}$$

dans laquelle $R_{10}$ représente un groupe divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :

a) dans les proportions de 60 à 100 % en moles, le groupe

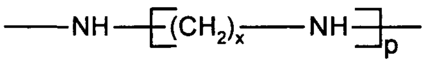

(IV)

où x=2 et p=2 ou 3, ou bien x=3 et p=2
ce groupe dérivant de la diéthylène-triamine, de la triéthylène-tétraamine ou de la dipropylène-triamine;
b) dans les proportions de 0 à 40 % en moles, le groupe (IV) ci-dessus, dans lequel x=2 et p=1 et qui dérive de l'éthylène-diamine,
ou le groupe dérivant de la pipérazine :

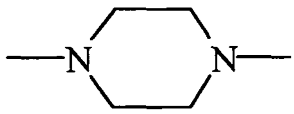

c) dans les proportions de 0 à 20 % en moles, le groupe $-NH-(CH_2)_6-NH-$ dérivant de l'hexaméthylènediamine, ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.

Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
Les alcane-sultones utilisées dans l'acylation sont de préférence la propane- ou la butane-sultone, les sels des agents d'acylation sont de préférence les sels de sodium ou de potassium.
(4) les polymères comportant des motifs zwittérioniques de formule :

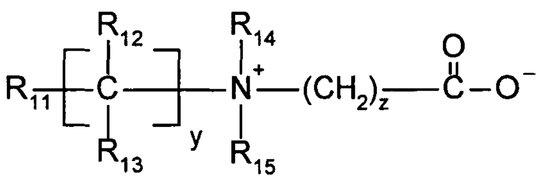

(IV)

dans laquelle $R_{11}$ désigne un groupement insaturé polymérisable tel qu'un groupement acrylate, méthacrylate, acrylamide ou méthacrylamide, y et z représentent un nombre entier de 1 à 3, $R_{12}$ et $R_{13}$ représentent un atome d'hydrogène, un groupe méthyle, éthyle ou propyle, $R_{14}$ et $R_{15}$ représentent un atome d'hydrogène ou un groupe alkyle de telle façon que la somme des atomes de carbone dans $R_{14}$ et $R_{15}$ ne dépasse pas 10.
Les polymères comprenant de tels motifs peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
A titre d'exemple, on peut citer les copolymères méthacrylate de méthyle/diméthyl-carboxyméthylammonio-éthylméthacrylate de méthyle, tels que le produit vendu sous la dénomination DIAFORMER Z301 par la société SANDOZ.
(5) les polymères dérivés du chitosane comportant des motifs monomères répondant aux formules suivantes :

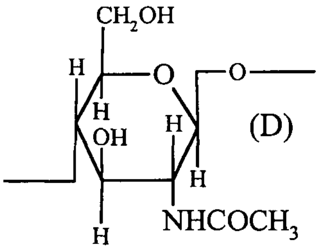
(D)

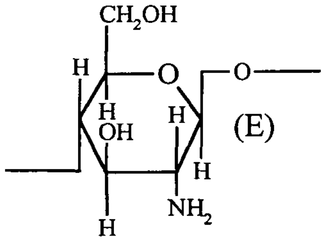
(E)

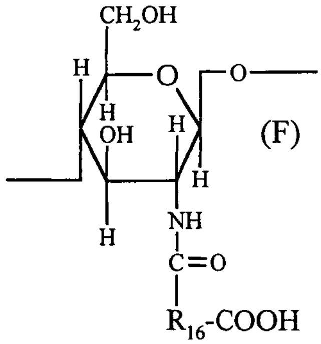
(F)

le motif (D) étant présent dans des proportions comprises entre 0 et 30%, le motif (E) dans des proportions comprises entre 5 et 50% et le motif (F) dans des proportions comprises entre 30 et 90%, étant entendu que dans ce motif (F), $R_{16}$ représente un groupe de formule :

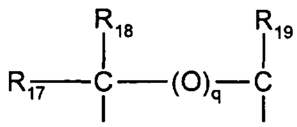

dans laquelle si q=0, $R_{17}$, $R_{18}$ et $R_{19}$, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes $R_{17}$, $R_{18}$ et $R_{19}$ étant dans ce cas un atome d'hydrogène ;
ou si q=1, $R_{17}$, $R_{18}$ et $R_{19}$ représentent chacun un atome d'hydrogène, ainsi que les sels formés par ces composés avec des bases ou des acides.
(6) Les polymères répondant à la formule générale (V) sont, par exemple, décrits dans le brevet français 1 400 366 :

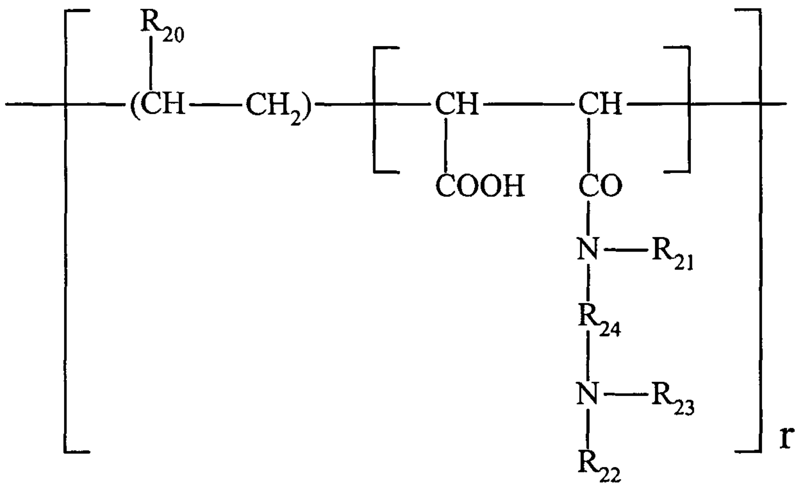
(V)

dans laquelle $R_{20}$ représente un atome d'hydrogène, un groupe $CH_3O$, $CH_3CH_2O$, phényle, $R_{21}$ désigne un atome d'hydrogène ou un groupe alkyle inférieur tel que méthyle, éthyle, $R_{22}$ désigne un atome d'hydrogène ou un groupe alkyle inférieur en $C_1$-$C_6$ tel que méthyle, éthyle, $R_{23}$ désigne un groupe alkyle inférieur en $C_1$-$C_6$ tel que méthyle, éthyle ou un groupe répondant à la formule : $-R_{24}-N(R_{22})_2$, $R_{24}$ représentant un groupement $-CH_2-CH_2-$, $-CH_2-CH_2-CH_2-$, $-CH_2-CH(CH_3)-$, $R_{22}$ ayant les significations mentionnées ci-dessus.
(7) Les polymères dérivés de la N-carboxyalkylation du chitosane comme le N-carboxyméthyl-chitosane ou le N-carboxybutyl-chitosane, vendu sous la dénomination "EVALSAN" par la société JAN DEKKER.

(8) Les polymères amphotères du type -D-X-D-X choisis parmi:

a) les polymères obtenus par action de l'acide chloracétique ou le chloracétate de sodium sur les composés comportant au moins un motif de formule :

-D-X-D-X-D- (VI)

où D désigne un groupe

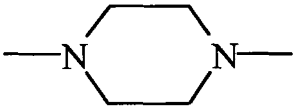

et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.

b) Les polymères de formule :

-D-X-D-X- (VI')

où D désigne un groupe

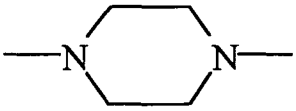

et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

(9) les copolymères alkyl($C_1$-$C_5$)vinyléther/anhydride maléique modifiés partiellement par semiamidification avec une N,N-dialkylaminoalkylamine telle que la N,N-diméthylaminopropylamine ou par semiestérification avec un N, N-dialkylaminoalcanol. Ces copolymères peuvent également comporter d'autres comonomères vinyliques tels que le vinylcaprolactame.

Parmi les polymères fixants additionnels amphotères décrits ci-dessus, les plus particulièrement préférés selon l'invention sont ceux de la famille (3) tels que les copolymères dont la dénomination CTFA est Octylacrylamide/ acrylates/ butylamino-ethylmethacrylate copolymer, tels que les produits vendus sous les dénominations AMPHOMER®, AMPHOMER® LV 71 ou LOVOCRYL® 47 par la société NATIONAL STARCH et ceux de la famille (4) tels que les copolymères méthacrylate de méthyle/diméthyl-carboxyméthylammonio-éthylméthacrylate de méthyle vendu par exemple sous la dénomination DIAFORMER® Z301 par la société SANDOZ.

Les polymères fixants additionnels non ioniques utilisables selon la présente invention sont choisis, par exemple, parmi :

- les polyalkyloxazolines ;

- les homopolymères d'acétate de vinyle ;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations PRIMAL® AC-261 K et EUDRAGIT® NE 30 D, par la société BASF sous la dénomination 8845, par la société HOECHST sous la dénomination APPRETAN® N9212 ;
- les copolymères d'acrylonitrile et d'un monomère non ionique choisis, par exemple, parmi le butadiène et les (méth) acrylates d'alkyle ; on peut citer les produits proposés sous la dénomination CJ 0601 B par la société ROHM & HAAS ;
- les homopolymères de styrène ;
- les copolymères de styrène comme, par exemple, les copolymères de styrène et de (méth)acrylate d'alkyle tels que les produits MOWILITH® LDM 6911, MOWILITH® DM 611 et MOWILITH® LDM 6070 proposés par la société HOECHST, les produits RHODOPAS® SD 215 et RHODOPAS® DS 910 proposés par la société RHONE POULENC ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame différents des homopolymères de vinylpyrrolidone, tels que le polyvinylcaprolactame commercialisé sous la dénomination Luviskol® PLUS par la société BASF ; et
- les copolymères de vinyllactame tels qu'un copolymère poly(vinylpyrrolidone/vinyllactame) vendu sous le nom commercial Luvitec® VPC 55K65W par la société BASF, les copolymères poly(vinylpyrrolidone/acétate de vinyle) comme ceux commercialisés sous la dénomination PVPVA® S630L par la société ISP, Luviskol® VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol® VAP 343 par la société BASF.

Les groupes alkyle des polymères non ioniques mentionnés ci-dessus ont, de préférence, de 1 à 6 atomes de carbone.

Selon l'invention, on peut également utiliser des polymères fixants additionnels de type siliconés greffés, comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0 582 152 et WO 93/23009 et les brevets US 4,693,935, US 4,728,571 et US 4,972,037.

Ces polymères peuvent être amphotères, anioniques ou non ioniques, et ils sont de préférence anioniques ou non ioniques.

De tels polymères sont, par exemple, les copolymères susceptibles d'être obtenus par polymérisation radicalaire à partir du mélange de monomères formé :

a) de 50 à 90 % en poids d'acrylate de tertiobutyle,
b) de 0 à 40 % en poids d'acide acrylique,
c) de 5 à 40 % en poids d'un macromère siliconé de formule

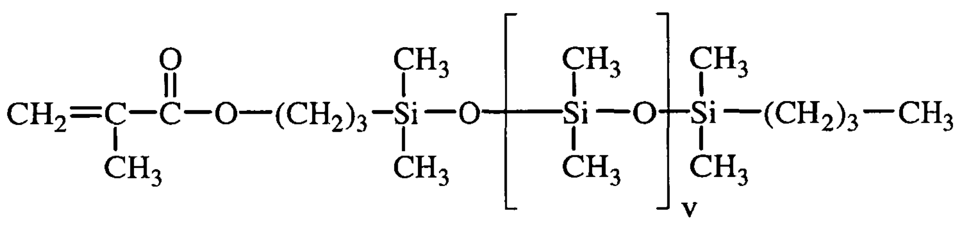

où v est un nombre allant de 5 à 700, les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type poly(acide (méth)acrylique) et du type poly((méth)acrylate d'alkyle), et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

On peut également utiliser comme polymères additionnels fixants, des polyuréthanes fonctionnalisés ou non,

non siliconés, cationiques, non-ioniques, anioniques ou amphotères, ou leurs mélanges.

Les polyuréthanes particulièrement visés par la présente invention sont ceux décrits dans les demandes EP 0 751 162, EP 0 637 600, EP 0 648 485 et FR 2 743 297 dont la demanderesse est titulaire, ainsi que dans les demandes EP 0 656 021 et WO 94/03510 de la société BASF, et EP 0 619 111 de la société National Starch.

Comme polyuréthanes convenant particulièrement bien dans la présente invention, on peut citer le produit commercialisé sous la dénomination LUVISET PUR® par la société BASF.

Le milieu cosmétiquement acceptable est de préférence constitué par de l'eau ou par un ou plusieurs solvants cosmétiquement acceptables tels que des alcools ou par des mélanges eau-solvant(s). Ces solvants sont de préférence des alcools en C1-C4.

Parmi ces alcools, on peut citer l'éthanol, l'isopropanol, l'éthanol étant particulièrement préféré.

La composition selon l'invention peut contenir en outre au moins un additif choisi parmi les tensioactifs non ioniques, anioniques, cationiques, amphotères ou zwitterioniques, les parfums, les filtres, les conservateurs, les protéines, les vitamines, les polymères non ioniques, anioniques, cationiques, amphotères ou zwitterioniques autres les polymères fixants décrits ci-dessus, les huiles minérales, végétales ou synthétiques, les agents et tout autre additif classiquement utilisé dans les compositions cosmétiques, tels que les agents antipelliculaires, les agents anti-chute, les colorants, les pigments, les agents hydratants tels que la glycérine et les autres polyols, les réducteurs.

Ces additifs sont présents dans la composition à des teneurs avantageusement comprises entre 0,001 et 20% en poids du poids total de la composition. La quantité précise de chaque additif est fonction de sa nature et est déterminée facilement par l'homme du métier, et dépendra de l'application capillaire choisie.

Bien entendu, l'homme du métier veillera à choisir le ou les additifs de manière que les propriétés avantageuses de la composition conforme à l'invention ne soient pas, ou substantiellement pas, altérées par l'addition envisagée.

Les compositions de l'invention peuvent être utilisées pour la fabrication de nombreux produits capillaires, comme par exemple, des produits pour la fixation et/ou le maintien des cheveux, des produits de conditionnement, comme des formulations de brillance ou encore des produits de soin des cheveux.

Ces compositions peuvent être conditionnées sous diverses formes, notamment dans des flacons pompes ou dans des récipients aérosols, afin d'assurer une application de composition sous forme vaporisée ou sous forme de mousse. De telles formes de conditionnement sont indiquées, par exemple, lorsque l'on souhaite obtenir un spray, une laque, ou une mousse pour la fixation ou le traitement des cheveux. Les compositions conformes à l'invention peuvent également se présenter sous la forme de crèmes, de gels, d'émulsions ou de lotions.

Lorsque la composition selon l'invention est conditionnée sous forme d'aérosol en vue d'obtenir une laque ou une mousse, elle comprend au moins un agent propulseur qui peut être choisi parmi les hydrocarbures volatils tels que le n-butane, le propane, l'isobutane, le pentane, un hydrocarbure halogéné et leurs mélanges. On peut également utiliser en tant qu'agent propulseur le gaz carbonique, le protoxyde d'azote, le diméthyléther (DME), l'azote, l'air comprimé. On peut aussi utiliser des mélanges de propulseurs. De préférence, on utilise le diméthyléther.

Avantageusement, l'agent propulseur est présent à une concentration comprise entre 5 et 90% en poids par rapport au poids total au poids total de la composition dans le dispositif aérosol, et plus particulièrement à une concentration comprise entre 10 et 60%.

Les exemples suivants sont destinés à illustrer l'invention.

**Exemple 1**

On applique la composition A suivante sur une mèche de cheveux naturels eurochâtain de 5 grammes.

Composition A :

| | | | |
|---|---|---|---|
| Permethyl 102 A | (1) | | 4% |
| Luviset Si PUR 2 | (2) | | 5% |
| Ethanol | | qsp | 100% |

(1) isoeicosane commercialisé par la société BAYER

(2) POLYURETHANE 6 (copolymère acide isophtalique/acide adipique/hexylèneglycol/néopentylglycol/ acide diméthoylolpropanoique /isophorone diisocyanate/bis-éthylaminoisobutyl-diméthicone), commercialisé par la société BASF.

La composition A est appliquée à l'aide d'un flacon pompe sur des cheveux naturels. Après séchage, on observe que la mèche possède un bon maintien et est brillante, douce au toucher, et d'un aspect agréable.

**Exemple 2**

On applique la composition B suivante sur une mèche de cheveux naturels eurochâtain de 5 grammes.

| Composition B : | | | |
|---|---|---|---|
| Permethyl 102 A | (1) | | 10% |
| DC 245 fluid | (2) | | 5% |
| DC 2-5225 C | (3) | | 7% |
| Luviset Si PUR | (4) | | 10% |
| Eau | | qsp | 100% |

(1) isoeicosane commercialisé par la société BAYER
(2) cyclopentadiméthylsiloxane commercialisé par la société DOW CORNING
(3) polydiméthyl/méthylsiloxane oxyéthyléné oxypropyléné commercialisé par la société DOW CORNING
(4) POLYURETHANE 6 (copolymère acide isophtalique/acide adipique/hexylèneglycol/néopentylglycol/ acide diméthoylolpropanoique /isophorone diisocyanate/bis-éthylaminoisobutyl-diméthicone), commercialisé par la société BASF.

La composition B est appliquée sur des cheveux naturels à l'aide d'un flacon pompe. Après séchage, on observe que la mèche possède un bon maintien, est brillante, douce au toucher et d'un aspect agréable.

**Revendications**

1. Composition cosmétique capillaire, **caractérisée en ce qu**'elle comprend dans un milieu cosmétiquement acceptable, de l'isoeicosane et au moins un polyuréthane fixant siliconé.

2. Composition selon la revendication 1, **caractérisée en ce que** l'isoeicosane est présent dans la composition à des teneurs comprises entre 0,05 et 20% en poids du poids total de la composition.

3. Composition selon la revendication 2, **caractérisée en ce que** l'isoeicosane est présent dans la composition à des teneurs comprises entre 1 et 10% en poids du poids total de la composition.

4. Composition selon l'une quelconque des revendications précédentes, **caractérisée en ce que** le polyuréthane fixant siliconé est choisi parmi les polyuréthanes fixants siliconés cationiques, anioniques, amphotères, non ioniques et leurs mélanges.

5. Composition selon l'une quelconque des revendications précédentes, **caractérisée en ce que** le polyuréthane fixant siliconé comprend un motif répétitif de base répondant à la formule générale (II) :

   $$- O - P - O - CO - NH - R - NH - CO - \qquad (II)$$

   dans laquelle :

   - P est un segment polysiloxanique, et
   - R est un groupe divalent choisi parmi les groupes alkylènes de type aromatique, aliphatique en $C_1$ à $C_{20}$, cycloaliphatique en $C_1$ à $C_{20}$, ces groupes étant substitués ou non.

6. Composition selon la revendication 5, **caractérisée en ce que** le polyuréthane fixant est un copolymère acide diméthylol-propionique/isophorone-diisocyanate/néopentylglycol/ polyesterdiols/diamine siliconé.

7. Composition selon l'une quelconque des revendications précédentes, **caractérisée en ce que** le polyuréthane

fixant siliconé est présent dans la composition à des teneurs comprises entre 0,01 et 20% en poids du poids total de la composition.

8. Composition selon la revendication 7, **caractérisée en ce que** le polyuréthane fixant siliconé est présent dans la composition à des teneurs comprises entre 0,05 et 15% en poids du poids total de la composition.

9. Composition selon la revendication 7, **caractérisée en ce que** le polyuréthane fixant siliconé est présent dans la composition à des teneurs comprises entre 0,1 et 10% en poids du poids total de la composition.

10. Composition selon l'une quelconque des revendications précédentes, **caractérisée en ce qu**'elle comprend en outre un ou plusieurs polymères fixants additionnels.

11. Composition selon l'une quelconque des revendications précédentes, **caractérisée en ce qu**'elle comprend en outre au moins un additif choisi parmi les tensioactifs non ioniques, anioniques, cationiques, amphotères ou zwitterioniques, les parfums, les filtres, les conservateurs, les protéines, les vitamines, les polymères non ioniques, anioniques, cationiques ou zwitterioniques, autres que les polymères fixants figurant dans les revendications précédentes, les huiles minérales, végétales ou synthétiques, les agents et tout autre additif classiquement utilisé dans les compositions cosmétiques, les agents anti-pelliculaires, les agents anti-chute, les colorants, les pigments, les agents hydratants, les réducteurs.

12. Procédé cosmétique capillaire, **caractérisé en ce qu**'il comprend l'application sur les cheveux d'une composition selon l'une quelconque des revendications 1 à 11.

13. Utilisation d'une composition selon l'une quelconque des revendications 1 à 11 pour conférer de la brillance aux cheveux.

## Claims

1. Cosmetic hair composition, **characterized in that** it comprises, in a cosmetically acceptable medium, isoeicosane and at least one silicone fixing polyurethane.

2. Composition according to Claim 1, **characterized in that** the isoeicosane is present in the composition at contents of between 0.05 and 20% by weight of the total weight of the composition.

3. Composition according to Claim 2, **characterized in that** the isoeicosane is present in the composition at contents of between 1 and 10% by weight of the total weight of the composition.

4. Composition according to any one of the preceding claims, **characterized in that** the silicone fixing polyurethane is chosen from cationic, anionic, amphoteric or nonionic silicone fixing polyurethanes, and mixtures thereof.

5. Composition according to any one of the preceding claims, **characterized in that** the silicone fixing polyurethane comprises a repeating base unit corresponding to general formula (II):

$$- O - P - O - CO - NH - R - NH - CO - \qquad (II)$$

in which:

- P is a polysiloxane segment, and
- R is a divalent group chosen from alkylene groups of aromatic type, of $C_1$ to $C_{20}$ aliphatic type and of $C_1$ to $C_{20}$ cycloaliphatic type, these groups possibly being substituted.

6. Composition according to Claim 5, **characterized in that** the fixing polyurethane is a dimethylol-propionic acid/isophorone diisocyanate/neopentyl glycol/polyesterdiols/diamine silicone copolymer.

7. Composition according to any one of the preceding claims, **characterized in that** the silicone fixing polyurethane is present in the composition at contents of between 0.01 and 20% by weight of the total weight of the composition.

**8.** Composition according to Claim 7, **characterized in that** the silicone fixing polyurethane is present in the composition at contents of between 0.05 and 15% by weight of the total weight of the composition.

**9.** Composition according to Claim 7, **characterized in that** the silicone fixing polyurethane is present in the composition at contents of between 0.1 and 10% by weight of the total weight of the composition.

**10.** Composition according to any one of the preceding claims, **characterized in that** it also comprises one or more additional fixing polymers.

**11.** Composition according to any one of the preceding claims, **characterized in that** it also comprises an additive chosen from nonionic, anionic, cationic, amphoteric or zwitterionic surfactants, fragrances, screening agents, preserving agents, proteins, vitamins, nonionic, anionic, cationic or zwitterionic polymers other than the fixing polymers which appear in the preceding claims, mineral, plant or synthetic oils, agents and any other additive conventionally used in cosmetic compositions, anti-dandruff agents, anti-hairloss agents, dyes, pigments, moisturizers and reducing agents.

**12.** Cosmetic hair process, **characterized in that** it comprises applying a composition according to any one of Claims 1 to 11 to the hair.

**13.** Use of a composition according to any one of Claims 1 to 11, for giving the hair a sheen.

## Patentansprüche

**1.** Haarkosmetische Zusammensetzung, **dadurch gekennzeichnet, dass** sie in einem kosmetisch akzeptablen Medium Isoeicosan und mindestens ein siliconiertes fixierendes Polyurethan enthält.

**2.** Zusammensetzung nach Anspruch 1, **dadurch gekennzeichnet, dass** das Isoeicosan in der Zusammensetzung in Mengenanteilen zwischen 0,05 und 20 Gew.-% des Gesamtgewichts der Zusammensetzung enthalten ist.

**3.** Zusammensetzung nach Anspruch 2, **dadurch gekennzeichnet, dass** das Isoeicosan in der Zusammensetzung in Mengenanteilen zwischen 1 und 10 Gew.-% des Gesamtgewichts der Zusammensetzung enthalten ist.

**4.** Zusammensetzung nach einem der vorhergehenden Ansprüche, **dadurch gekennzeichnet, dass** das siliconierte fixierende Polyurethan unter den kationischen, anionischen, amphoteren oder nichtionischen, siliconierten fixierenden Polyurethanen und deren Gemischen ausgewählt ist.

**5.** Zusammensetzung nach einem der vorhergehenden Ansprüche, **dadurch gekennzeichnet, dass** das siliconierte fixierende Polyurethan eine wiederkehrende Grundeinheit der folgenden Formel (II) enthält:

-O-P-O-CO-NH-R-NH-CO- (II)

worin bedeuten:

- P ein Polysiloxansegment, und
- R eine zweiwertige Gruppe, die unter den Alkylengruppen vom aromatischen Typ, aliphatischen Typ mit 1 bis 20 Kohlenstoffatomen und cycloaliphatischen Typ mit 1 bis 20 Kohlenstoffatomen ausgewählt ist, wobei diese Gruppen substituiert oder unsubstituiert sind.

**6.** Zusammensetzung nach Anspruch 5, **dadurch gekennzeichnet, dass** das fixierende Polyurethan ein siliconiertes Dimethylolpropionsäure / Isophorondüsocyanat/ Neopentylglycol/ Polyesterdiole/Diamin- Copolymer ist.

**7.** Zusammensetzung nach einem der vorhergehenden Ansprüche, **dadurch gekennzeichnet, dass** das siliconierte fixierende Polyurethan in der Zusammensetzung in Mengenanteilen zwischen 0,01 und 20 Gew.-% des Gesamtgewichts der Zusammensetzung enthalten ist.

**8.** Zusammensetzung nach Anspruch 7, **dadurch gekennzeichnet, dass** das siliconierte fixierende Polyurethan in der Zusammensetzung in Mengenanteilen zwischen 0,05 und 15 Gew.-% des Gesamtgewichts der Zusammensetzung enthalten ist.

**9.** Zusammensetzung nach Anspruch 7, **dadurch gekennzeichnet, dass** das siliconierte fixierende Polyurethan in der Zusammensetzung in Mengenanteilen zwischen 0,1 und 10 Gew.-% des Gesamtgewichts der Zusammensetzung enthalten ist.

**10.** Zusammensetzung nach einem der vorhergehenden Ansprüche, **dadurch gekennzeichnet, dass** sie ferner ein oder mehrere zusätzliche fixierende Polymere enthält.

**11.** Zusammensetzung nach einem der vorhergehenden Ansprüche, **dadurch gekennzeichnet, dass** sie ferner mindestens einen Zusatzstoff enthält, der unter den nichtionischen, anionischen, kationischen, amphoteren oder zwitterionischen grenzflächenaktiven Stoffen, Parfums, Filtern, Konservierungsmitteln, Proteinen, Vitaminen, nichtionischen, anionischen, kationischen oder zwitterionischen Polymeren, die von den in den vorhergehenden Ansprüchen beschriebenen fixierenden Polymeren verschieden sind, Mineralölen, pflanzlichen Ölen oder synthetischen Ölen, Mitteln und allen anderen Zusatzstoffen, die gewöhnlich in kosmetischen Zusammensetzungen verwendet werden, Antischuppenmitteln, Wirkstoffen gegen Haarausfall, Farbstoffen, Pigmenten, Hydratisierungsmitteln und Reduktionsmitteln ausgewählt ist.

**12.** Kosmetisches Verfahren für die Haarbehandlung, **dadurch gekennzeichnet, dass** es umfasst, eine Zusammensetzung nach einem der Ansprüche 1 bis 11 auf die Haare aufzutragen.

**13.** Verwendung einer Zusammensetzung nach einem der Ansprüche 1 bis 11, um den Haaren Glanz zu verleihen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2815853
- FR 2708199
- FR 2743297
- EP 080976 A
- FR 2077143
- FR 2393573
- US 4131576 A
- FR 1222944
- DE 2330956
- FR 1580545
- FR 2265782
- FR 2265781
- FR 1564110
- FR 2439798
- GB 2047398 A
- GB 2723248 A
- GB 2102113 A
- GB 839805 A
- FR 2350384
- FR 2357241
- FR 2198719
- US 4128631 A
- FR 1400366
- EP 0412704 A
- EP 0412707 A
- EP 0640105 A
- WO 9500578 A
- EP 0582152 A
- WO 9323009 A
- US 4693935 A
- US 4728571 A
- US 4972037 A
- EP 0751162 A
- EP 0637600 A
- EP 0648485 A
- EP 0656021 A
- WO 9403510 A
- EP 0619111 A